# EUROPEAN PATENT SPECIFICATION

(11) **EP 2 149 332 B1**
(45) Date of publication and mention of the grant of the patent: **17.12.2014**
(21) Application number: 08751662.1
(22) Date of filing: 01.05.2008
(51) Int. Cl.: A61B 1/04, G06T 1/00

(54) **IMAGE INFORMATION DISPLAY PROCESSING DEVICE AND DISPLAY PROCESSING METHOD**
VERARBEITUNGSVORRICHTUNG FÜR EINE BILDINFORMATIONSANZEIGE UND ANZEIGEVERARBEITUNGSVERFAHREN
DISPOSITIF DE TRAITEMENT DE L'AFFICHAGE D'INFORMATIONS D'IMAGES ET PROCÉDÉ DE TRAITEMENT DE L'AFFICHAGE

(30) Priority: 17.05.2007 JP 2007131764
(43) Date of publication of application: 03.02.2010
(73) Proprietor: Olympus Medical Systems Corp., Tokyo 151-0072 (JP)
(72) Inventor: YOKOYAMA, Eri, Tokyo 151-0072 (JP)
(74) Representative: Gunzelmann, Rainer
(86) International application number: PCT/JP2008/001137
(87) International publication number: WO 2008/142831

(56) References cited:
- EP-A1- 1 618 828
- WO-A1-2005/031650
- JP-A- 2004 337 596
- JP-A- 2006 138 957
- JP-A- 2006 334 297
- JP-A- 2006 334 297
- JP-A- 2006 345 929
- JP-A- 2006 345 929
- JP-A- 2006 346 356
- US-A1- 2007 060 798

## Description

### Technical Field

The present invention relates to a display processing apparatus and display processing method of image information for displaying plural pieces of image information obtained by capturing images with time by moving, for example, a capsule endoscope with autonomy or heteronomy.

### Background Art

Recently, in an endoscope field, an endoscope to be swallowed, that is, a capsule endoscope, has been developed. As the conventional technology relating to the capsule endoscope, the specification of the patent document 1 discloses the technology of having an image capturing function and a wireless communication function, sequentially capturing an organ such as a stomach, a small intestine, etc. for an observation period after a patient swallows an endoscope for an observation or an inspection from the mouth until the endoscope is naturally discharged from the human body, and sequentially transmitting image information (electronic data representing an image) by capturing images.

Furthermore the patent document 2 discloses the technology of receiving the image information transmitted by wireless as described above by a receiver provided outside the patient and accumulating in predetermined memory, and afterwards reading the information as necessary and displaying the information on a display unit, thereby utilizing the information for a diagnosis etc. by a doctor.

However, with the above-mentioned capsule endoscope, unlike a normal endoscope, the period after a patient swallows from his or her mouth until it is naturally discharged is an observation period or an inspection period. Therefore, the observation period or the inspection period can require a long time, for example, ten or more hours, and there are a large number of pieces of image information obtained during the period by capturing image.

Thus, at the stage of a diagnosis etc., it is not easy to grasp the large number of pieces of image information for a short time, and it is not easy to detect the image information about a desired portion to be checked from among the large number of pieces of image information, to be more practical, it is not easy to detect the image information about a desired organ to be checked for a diagnosis, and the image information only relating to the images of affected parts.

EP 1 618 828 A1 discloses an image display apparatus with the aim of improving the ability to retrieve a taken image of an internal body and of recognizing the organ depicted in each image. The apparatus comprises the display of an average colour bar indicating the overall imaging period of images taken in time sequence by a capsule endoscope, an image displayed in an image display field, a movable slider superposed on the average colour bar, and a flag as a bleeding part displayed above and parallel to the average colour bar.

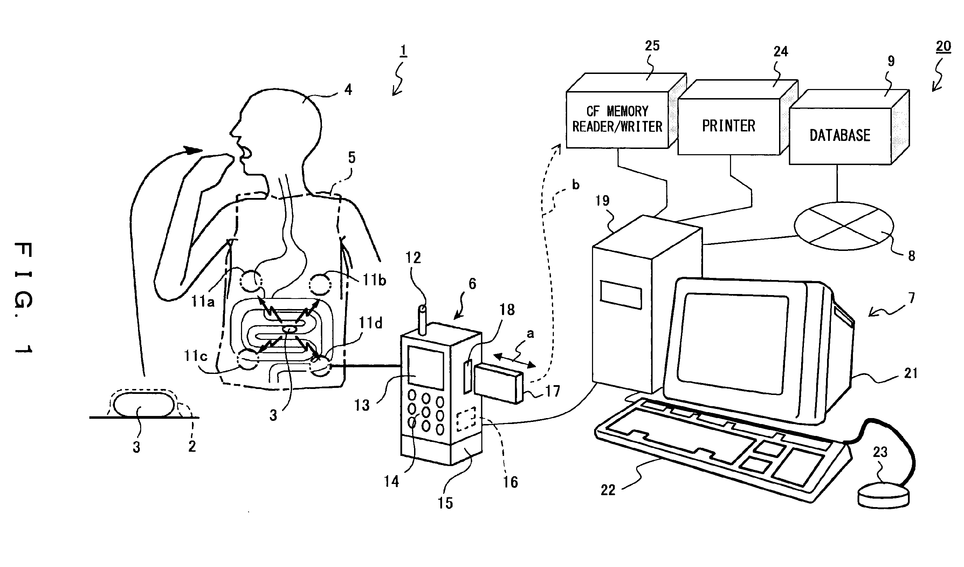

US 2007/060798 A1 and WO 2005/031650 A1 disclose an in-vivo sensing system with the aim of creating a summarized graphical presentation of a data stream captured in-vivo. The system comprises a colour bar serving as the graphical presentation and being a fixed display alongside a streaming display of the in-vivo data stream, and a cursor, icon or other indicator movable along the fixed colour bar and/or a time bar when the data stream is displayed and/or streamed. JP 2006-334297 and JP 2006-345929 disclose a similar system, wherein thumbnail images are displayed next to the image bar. The area for the display of observation images remains fixed, although the number of images displayed therein can vary.
Patent Document 1: Specification of U.S. Patent Application Laid-Open No. 2002/0177779A1
Patent Document 2: Japanese Published Patent Application No. 2005-218584 (abstract, FIGS. 1, 2, and 3)

### Disclosure of the Invention

An object of the present invention is to provide a display processing apparatus and a display processing method capable of easily grasping the image information about captured images of a desired position to be checked and affected parts etc. from a large number of pieces of image information, and capable of quickly displaying a large number of pieces of image information on a small display screen.

The display processing apparatus according to the present invention displays, on a display screen, image information about the video of plural pieces obtained by capturing with time the video in a plurality of positions in the body of a test subject using an image pickup apparatus provided in the body of the test subject. The apparatus includes: an image bar generation device adapted to arrange a line expressed in an average color calculated for each piece of video or for plural pieces of video on a display screen in a time series, and adapted to generate an image bar that can be totally displayed as a bar; a mark generation device adapted to generate a mark for displaying a feature determined from video as a feature extracted mark extracted from the video, and a mark display device adapted to display a mark generated by the mark generation device superposed on the image bar or separated in parallel above the image bar, characterized in that the mark display device is further adapted to switch the display of the mark from the display of the mark superposed and displayed on the image bar when an observation image is displayed, into the display of the mark separated in parallel above the image bar in response to a user input and in cooperation with a replacement of an area of the display screen where the observation image is displayed, and from the display of the mark separated in parallel above the image bar when the observation image is displayed, into the display of the mark superposed and displayed on the image bar when the user specifies a display of the observation image corresponding to an enlargement of the area of the display screen in which the observation image is displayed.

According to the present invention, since a mark in each color depending on the feature of video can be displayed as superposed on an image bar indicating captured image information in a time series, a display area of a display screen can be space-saved, thereby reserving a larger observation screen area.

In addition, since display can be switched in various input methods appropriate for the display being displayed, only necessary information can be selected and displayed quickly.

### Brief Description of Drawings

FIG. 1 shows the configuration of the outline of the capsule endoscope image filing system according to the present invention;

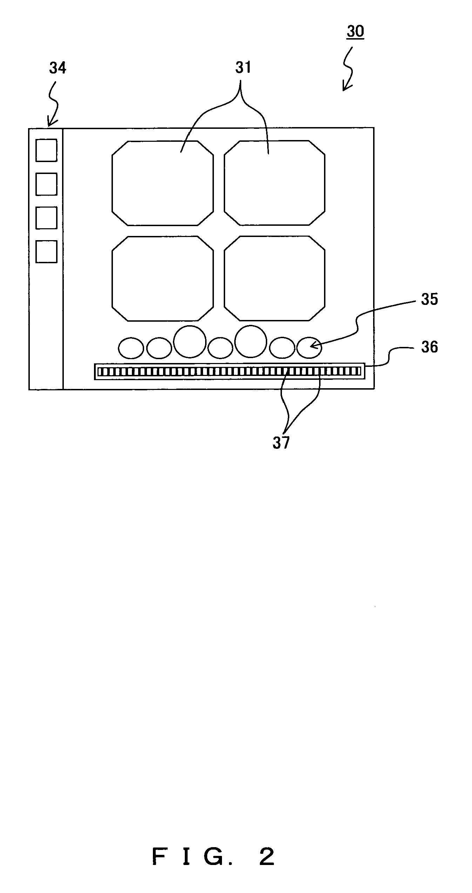

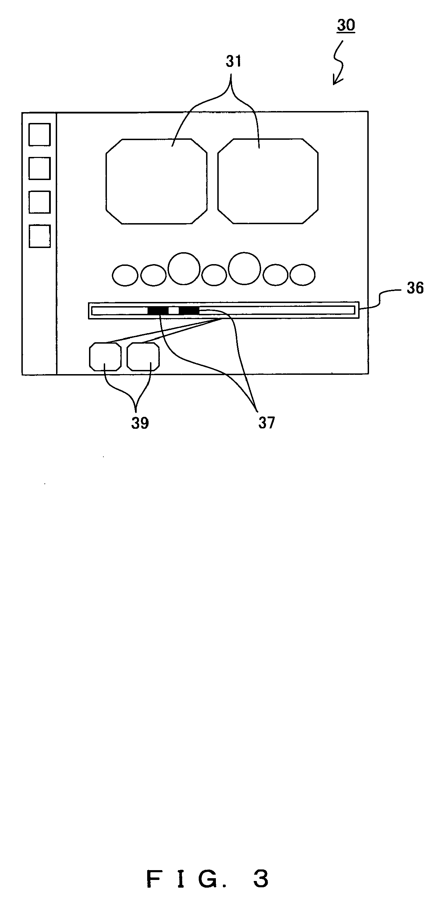

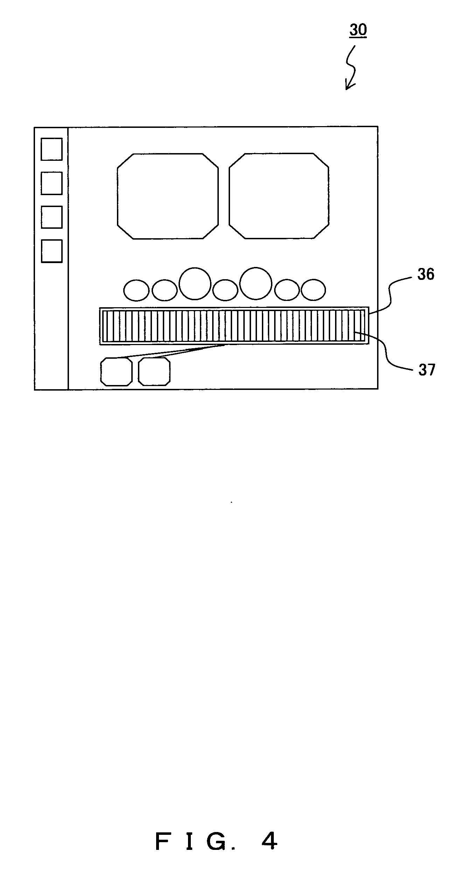

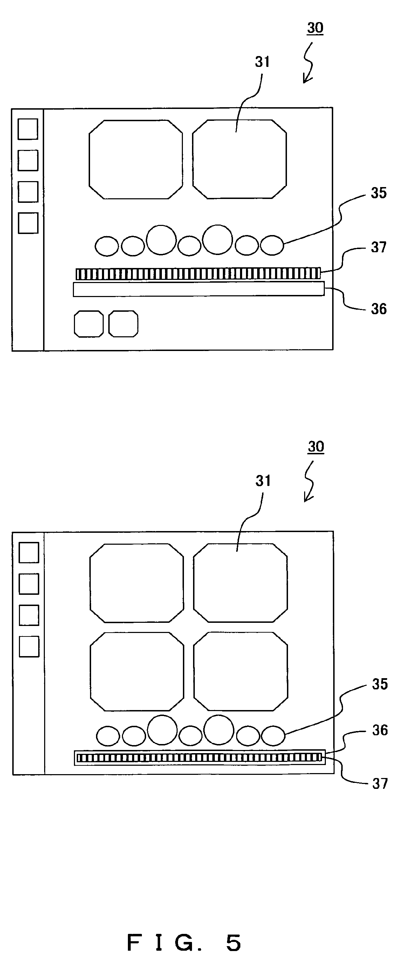

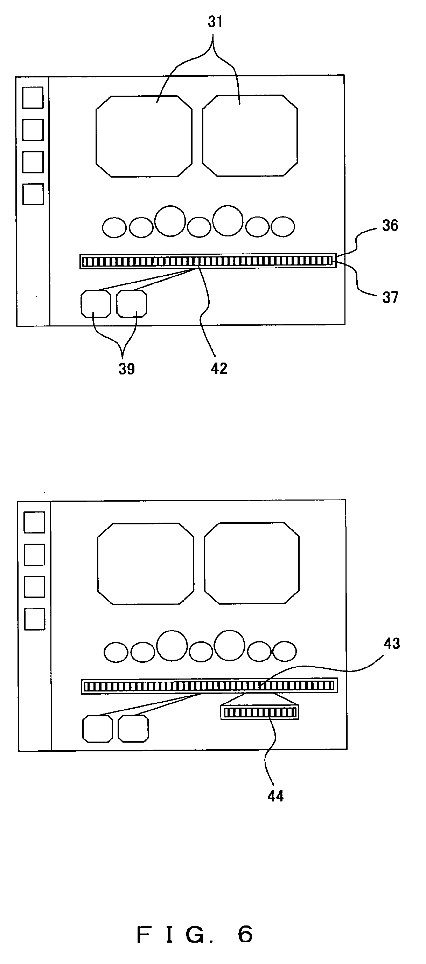

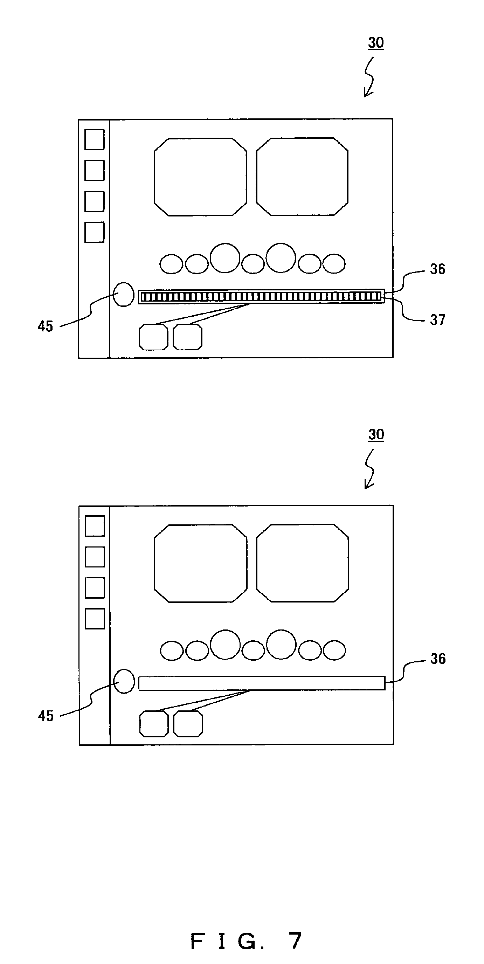

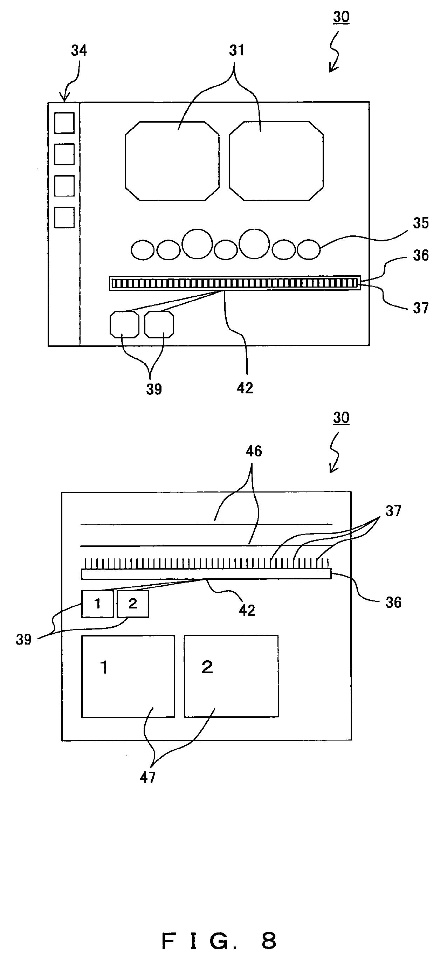

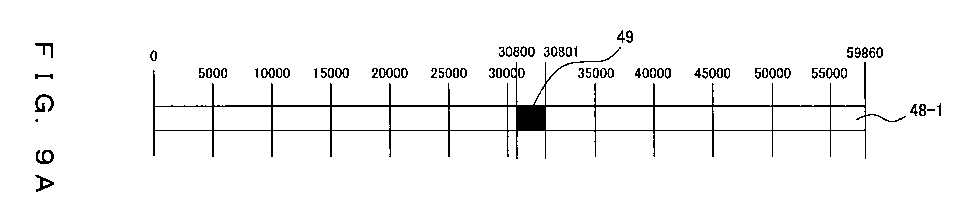

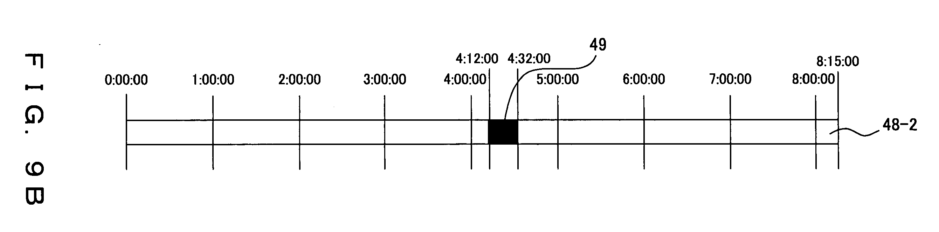

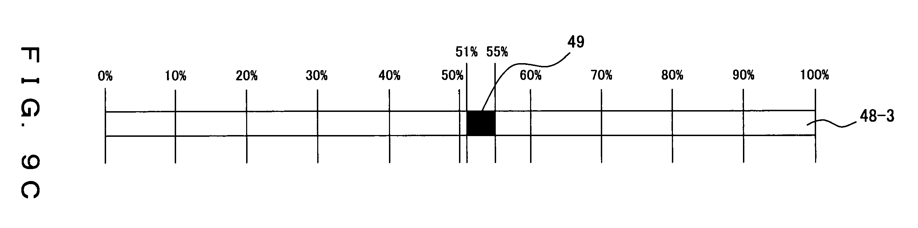

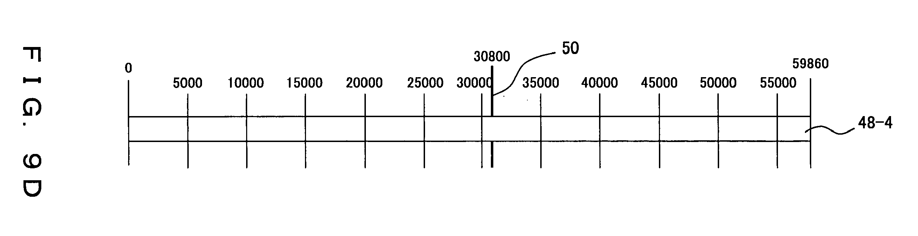

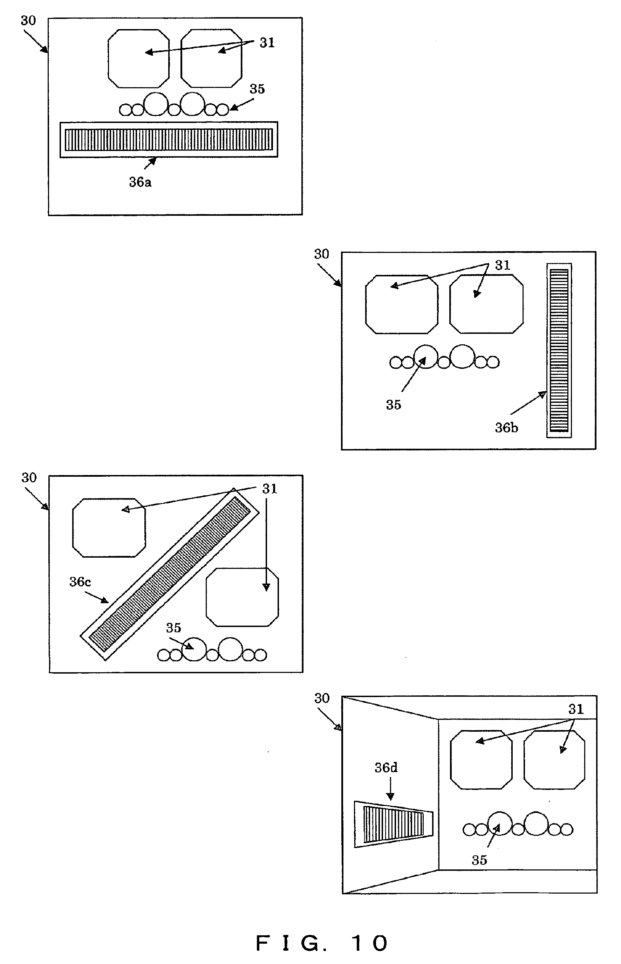

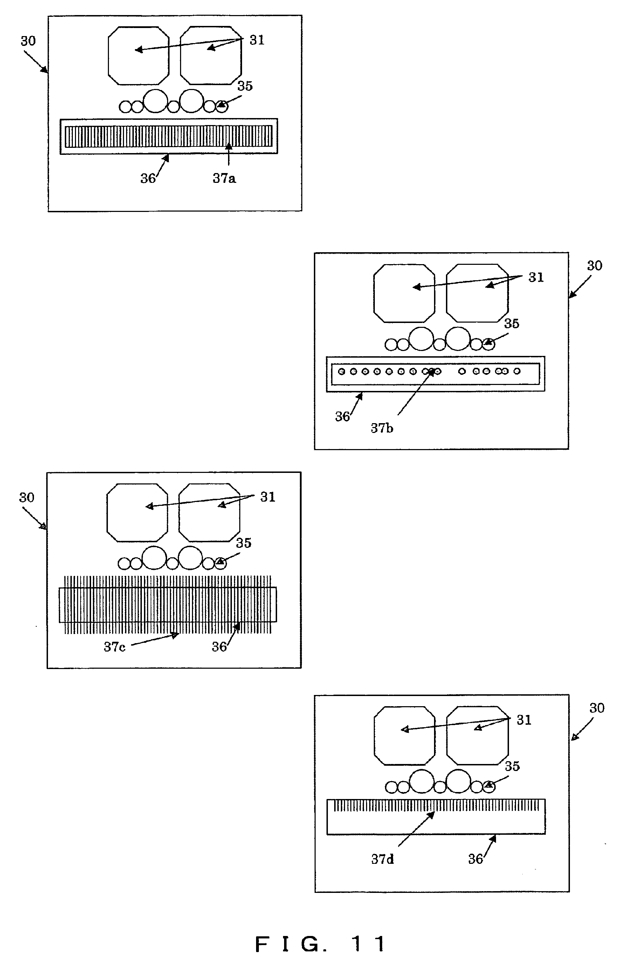

FIG. 2 shows an example of an image displayed on the display screen of a monitor device of a work station in the capsule endoscope image filing system as the first embodiment not according to the present invention; FIG. 3 shows the state of the marking of a vertical stripe as a red mark as another example of the first embodiment continuously spreading as a plane along the superposing display area of an image bar; FIG. 4 shows an example of an image displayed on the display screen of a monitor device of a work station in the capsule endoscope image filing system as the second embodiment not according to the present invention; FIG. 5 shows an example of an image displayed on the display screen of a monitor device of a work station in the capsule endoscope image filing system as the third embodiment of the present invention; FIG. 6 shows an example of an image displayed on the display screen of a monitor device of a work station in the capsule endoscope image filing system as the fourth embodiment not according to the present invention; FIG. 7 shows an example of an image displayed on the display screen of a monitor device of a work station in the capsule endoscope image filing system as the fifth embodiment not according to the present invention; FIG. 8 shows an example of an image displayed on the display screen of a monitor device of a work station in the capsule endoscope image filing system as the sixth embodiment of the present invention; FIG. 9A shows an example (1) of an image displayed on the display screen of a monitor device of a work station in the capsule endoscope image filing system as the seventh embodiment not according to the present invention; FIG. 9B shows an example (2) of an image displayed on the display screen of a monitor device of a work station in the capsule endoscope image filing system as the seventh embodiment not according to the present invention; FIG. 9C shows an example (3) of an image displayed on the display screen of a monitor device of a work station in the capsule endoscope image filing system as the seventh embodiment not according the present invention; FIG. 9D shows an illustration (4) of an image displayed on the display screen of a monitor device of a work station in the capsule endoscope image filing system as a seventh example; FIG. 10 shows an example expressing in a three-dimensional array in the horizontal, vertical, diagonal, or depth direction on the screen a vertical line as an example of a long image bar horizontally arranged in a time series; and FIG. 11 shows an example of expressing a variable line, dot, circle, square, vertical line longer or shorter than the width of the bar with respect to the horizontal bar.

### Explanation of Reference Numeral

1 capsule endoscope system 2 package 3 capsule endoscope 4 test subject 5 jacket 6 receiver 7 work station 8 network 9 database 11(11a, 11b, 11c, 11d) antenna
12 antenna
13 display unit
14 input unit
15 power supply unit
16 signal processing/controlling unit
17 CF (compact flash (registered trademark)) memory
18 attachment unit
19 body device
20 capsule endoscope image filing system
21 monitor device
22 keyboard
23 mouse
24 printer
25 CF/memory reader/writer
30 display screen
31 observation image
34 instruction button
35 reproduction button group
36((36a, 36b, 36c, 36d) image bar
37((37a, 37b, 37c, 37d) mark
39 thumbnail image
42 check point
43 target range
44 partial enlargement display
45 switch button
46 report entry area
47 report content
48-1, 48-2, 48-4 scale mark
49 gap
50 gap mark

### Best Mode for Carrying out the Invention

The embodiments of the present invention are described below in detail with reference to the attached drawings.

FIG. 1 shows the capsule endoscope system according to the present invention and the capsule endoscope image filing system included in the capsule endoscope system.

As shown in FIG. 1, a capsule endoscope system 1 according to the present embodiment includes a capsule endoscope 3 contained in a package 2; a patient who swallows the capsule endoscope 3 taken out of the package 2, that is, a test subject 4; a jacket 5 for the test subject 4; and a receiver 6 freely attached to and detached from the jacket 5.

A capsule endoscope image filing system 20 is configured by a work station 7 for storing and editing image data received by the receiver 6, and a database 9 connected to the work station 7 through a network 8. The database 9 can also be built in the work station 7.

The capsule endoscope 3 contains an image pickup unit, a radio unit and a power supply unit. In the period after the capsule endoscope 3 is swallowed by the test subject 4 for an observation or an inspection from his or her mouth until it is discharged from the body of the test subject, it transmits by wireless the image data obtained by sequentially capturing images of the organs such as the esophagus, the stomach, the small intestine, the large intestine, etc. of the test subject as radio waves from the radio unit to the outside the body of the test subject.

The jacket 5 clothed in the test subject 4 is provided with a plurality of (four in the example shown in FIG. 1) antennas 11 (11a, 11b, 11c, and 11d) for capturing emitted radio waves of image data emitted from the radio unit of the capsule endoscope 3. These antennas 11 can communicate with the receiver 6 by wireless or by cable.

In addition, the number of the antennas 11 is not limited to four, but can be appropriately determined. That is, any number appropriate for receiving radio waves emitted depending on the position with respect to the movement of the capsule endoscope 3 is acceptable.

The receiver 6 is externally provided with an antenna 12 for use when image data is received from the jacket 5 through the antenna 11, a display unit 13 for displaying necessary information for an observation or an inspection, and an input unit 14 for inputting necessary information for an observation or an inspection.

Then, a power supply unit 15 for providing power also for a portable unit is provided below the receiver 6. The power supply unit 15 is configured by, for example, a dry battery, a Li ion secondary battery, a Ni hydrogen battery, etc. (a battery in other formats can be acceptable).

Furthermore, in the receiver 6, a signal processing and controlling unit 16 for performing necessary processes for an observation or an inspection is provided, and an attachment unit 18 for attaching CF (CompactFlash (registered trademark)) memory 17 for storing received image data in such a way that the memory can be attached/detached as indicated by the bidirectional arrow a shown in FIG. 1.

The work station 7 is provided with a body device 19, a monitor device 21 connected to the body device 19, a keyboard 22, a mouse 23, etc. The body device 19 is provided with various interfaces in addition to an interface for connection to the network 8 although not shown in FIG. 1.

In addition to the above-mentioned receiver 6, a printer 24 and a CF memory reader/writer 25 are connected to the work station 7 through the interfaces.

The work station 7 has the image processing function for a doctor or a nurse performing a diagnosis etc. by displaying an image in the gut of the test subject 4 captured by the capsule endoscope 3.

While the doctor or the nurse are performing an inputting operation on a man-machine interface displayed on the monitor device 21 of the work station 7 using the keyboard 22 or the mouse 23, the doctor or the nurse can issue an instruction to fetch the image data about the inside of the gut of the test subject 4 transmitted from the package 2 and received by the receiver 6.

As to the fetch of the image data from the receiver 6, data can be directly fetched from the receiver 6, and input data can also be fetched from the CF memory 17 by attaching the CF memory 17 at the CF memory reader/writer 25 as indicated by the arrow b shown in FIG. 1.

Furthermore, the doctor or the nurse can issue an instruction to store the captured image screen data fetched from the receiver 6 as described above in the database 9, an instruction to display an image relating to the image data described later on the display screen of the monitor device 21 by calling the image data stored in the database 9, an instruction to record a diagnosis result etc. based on an observation of an image in the database 9, an instruction to print a card etc. on the printer 24, etc.

According to the present invention, the capsule endoscope system and a capsule endoscope image filing system is specifically described, but absolutely clear the present invention is not limited to these applications. (First Embodiment)

FIG. 2 shows an example of an image displayed on the display screen of a monitor device of a work station in the capsule endoscope image filing system as the first embodiment not according to the present invention.

As shown in FIG. 2, a display screen 30 displays four observation images 31. At the upper left of the display screen 30, a plurality of (four in the example shown in FIG. 2) instruction buttons 34 are displayed.

And displayed below the four observation images 31 is a reproduction button group 35 including a stop button at the center, a replay button, a fast forward button, and a frame advance button on the right, and the respective inverse buttons on the left. Displayed further below the buttons is a image bar 36, and a mark 37 are superposed on the image bar 36.

The image bar 36 shows as a vertical line an average color calculated for each captured image or a plurality of captured images at each position on a time axis when the capsule endoscope 3 moves in an organ. A vertical line is arranged in a time series horizontally on the display screen, and the entire vertical lines are represented as a horizontal bar.

That is, the representation of an average color by the image bar 36 is the image bar 36 as a horizontal arrangement on the display screen 30 by arranging a vertical line represented by an average color calculated for each captured image (video) or for a plurality of captured images along a movement time axis of the capsule endoscope 3, that is, in a time series, to roughly know the position of a captured image based on the characteristic of a different color for each organ.

Furthermore, the mark 37 is displayed by a red line superposed on the image bar 36 at a position corresponding to the time axis of the image bar 36 as a detection position obtained by detecting the characteristic determined from the video on a time axis, for example, red color indicating bleeding. That is, the mark 37 is a type of lesion color mark.

Such a lesion is not limited to bleeding, but can be another lesion confirmed by a captured image by the capsule endoscope 3. These lesions can also be displayed as other color marks than the red color mark such as blue, white, or other color marks.

Assuming that the display screen 30 is a normal display format, thumbnail images are display at the bottom row, or although there are no thumbnail images to be displayed, a lesion color bar and a time axis bar are arranged parallel to the image bar 36. Therefore, at most two observation images 31 can be displayed.

However, in the present embodiment shown in FIG. 2, the mark 37 indicating a lesion color is superposed and displayed on the display of the image bar 36. That is, in the present embodiment, since the information about a lesion etc. is display as a mark in the display area of the image bar 36, the display area of the observation image 31 of the display screen 30 can be expanded, thereby simultaneously displaying four currently selected observation images 31.

The mark 37 superposed and displayed on the display of the image bar 36 is displayed as red vertical lines (black vertical lines in FIG. 2) that are discontinuous vertical stripe markings. However, when a bleeding lesion portion is continuously arranged on the image pickup path of the capsule endoscope 3, the vertical stripe markings appear as continuous markings.

FIG. 3 shows the state of the marking of a vertical stripe of the mark 37 as another example of the first embodiment continuously spreading as a plane along the superposing display area of an image bar 36.

In FIG. 3, the display portion having the same function as in FIG. 2 is assigned the same reference numeral as in FIG. 2.

In the example shown in FIG. 3, there are two currently selected observation images 31, and thumbnail images 39 are display below the mark 37 superposed and displayed on the image bar 36.

In this case, the lower display area can be larger by the mark 37 superposed and displayed on the image bar 36, thereby displaying the thumbnail images 39 larger.

Furthermore, although the bar display area is large, the mark 37 is superposed and displayed on the image bar 36 to allow a user to recognize at a glance the position of an important marking of the mark 37 occurring in a time series of the image bar 36.

That is, when the image bar 36 and the mark 37 are separately displayed above and below with respect to each other, a user of the monitor device 21 has to move the view point up and down on the display screen of the display screen 30 to compare the position of the image bar 36 with the position of the mark 37 and confirm the time position of the mark 37.

As shown in FIG. 2 or 3, when the mark 37 is superposed and displayed on the image bar 36, a user can recognize the marking position of the mark 37 without moving the view point up and down.

In addition in FIG. 2 or 3, although it cannot be clearly shown, but a user can display the mark 37 as a semitransparent mark superposed and displayed on the image bar 36. Furthermore, the transparence of the semitransparent mark can be appropriately specified.

Thus, the color of the image bar 36 of the portion where the marking of the mark 37 appears can be known, thereby performing an appropriate diagnosis.

### (Second Embodiment)

FIG. 4 shows an example of an image displayed on the display screen of a monitor device of a work station in the capsule endoscope image filing system as the second embodiment not according to the present invention.

In FIG. 4, the display portion having the same function as in FIGS. 2 and 3 is assigned the same reference numeral as in FIGS. 2 and 3.

In the display on the display screen 30 according to the present embodiment shown in FIG. 4, the widths of the image bar 36 and the mark 37 superposed and displayed on the image bar 36 are expanded vertically on the screen. The vertical line of the mark 37 is elongated with the vertical expansion of the width on the screen of the mark 37.

It is a result of a change based on an external instruction input on the display screen 30, that is, by a user. Thus, the width of the image bar 36 and the mark 37 superposed and displayed on the image bar 36 can be changed into an arbitrary width.

Then, when the width of the image bar is expanded as shown in FIG. 4, the vertical resolution of the average color of one image (or a plurality of images) represented by one vertical line is enhanced, thereby displaying further detailed data.

This holds true also with the mark 37.

### (Third Embodiment)

FIG. 5 shows an example of an image displayed on the display screen of a monitor device of a work station in the capsule endoscope image filing system as the third embodiment of the present invention

In FIG. 5, the display portion having the same function as in FIGS. 2 through 4 is assigned the same reference numeral as in FIGS. 2 through 4.

In the display on the display screen 30 according to the present embodiment shown in FIG. 5, the mark 37 is arranged above the image bar 36 in parallel.

When a user specifies the display of the observation image 31 as displaying four images or enlarging and displaying an image, the display area is automatically narrowed below the 35, and the mark 37 display in parallel above the image bar 36 is superposed and displayed on the image bar 36.

Thus, the mark 37 can be changed to be superposed and displayed on the image bar 36, thereby reserving a broad display area for the observation image 31 without impairing the conditions of a comparing observation between the image bar 36 and the mark 37.

### (Fourth Embodiment)

FIG. 6 shows an example of an image displayed on the display screen of a monitor device of a work station in the capsule endoscope image filing system as the fourth embodiment not according to the present invention.

In FIG. 6, the display portion having the same function as in FIGS. 2 through 5 is assigned the same reference numeral as in FIGS. 2 through 5.

In the display on the display screen 30 according to the present embodiment shown in FIG. 6, the mark 37 is superposed and displayed on the mark 37 as shown in FIG. 6, and the two thumbnail images 39 and the two observation images 31 selected on the check point 42 by the user in the superposing display are displayed.

Thus, when there is an available area to the right of the display area of the thumbnail images 39 as shown in FIG. 6, a target area 43 in the superposed and displayed bar specified by the user is enlarged and can be separately displayed as a partial enlargement display 44 of the mark 37 superposed and displayed on the image bar 36 as shown at the lower portion in FIG. 6.

### (Fifth Embodiment)

FIG. 7 shows an example of an image displayed on the display screen of a monitor device of a work station in the capsule endoscope image filing system as the fifth embodiment not according to the present invention.

In FIG. 7, the display portion having the same function as in FIGS. 2 through 6 is assigned the same reference numeral as in FIGS. 2 through 6.

In the display on the display screen 30 according to the present embodiment shown in FIG. 7, a switch button 45 is displayed close to the leftmost portion of the mark 37 superposed and displayed on the image bar 36 as shown at the upper portion in FIG. 7.

As shown at the upper portion in FIG. 7, when the mark 37 is superposed and displayed on the image bar 36, and when the switch button 45 is pressed (clicked) by a pointing device such as a mouse etc., the display control unit determines that a no display instruction is input for the mark 37, and suppresses the display of the mark 37 superposed and displayed on the image bar 36 as shown at the lower part in FIG. 7.

On the other hand, as shown at the lower part in FIG. 7, when the mark 37 is not superposed and displayed on the image bar 36, and when the pointing device is clicked on the switch button 45, the display control unit determines that a display instruction is input for the mark 37, and superposes and displays the mark 37 on the image bar 36 as shown at the upper part in FIG. 7.

A "display/no-display" switch instruction can be specified for the image bar 36. In this case, when the display of the image bar 36 is cleared, the entire information about the mark 37 can be confirmed.

It is obvious that the "display/no-display" switch instruction is not limited to the clicking operation on the switch button 45 as described above, but the instruction can be specified by clicking the pointing device on a menu bar or a tool chip.

### (Sixth Embodiment)

FIG. 8 shows an example of an image displayed on the display screen of a monitor device of a work station in the capsule endoscope image filing system as the sixth embodiment of the present invention.

In FIG. 8, the display portion having the same function as in FIGS. 2 through 7 is assigned the same reference numeral as in FIGS. 2 through 7.

In the display on the display screen 30 according to the present embodiment shown in FIG. 8, it is assumed that a user sees a report of the observation image 31 or inputs an instruction to enter data in the report when the instruction buttons 34, the two observation images 31, the group 35 of seven reproduction buttons, the mark 37 superposed and displayed on the image bar 36, and the thumbnail images 39 corresponding to the observation image 31 are displayed at the upper part in FIG. 8.

Then, as shown at the lower part in FIG. 8, a report entry area 46 is displayed in the area where the observation image 31 is displayed, and furthermore the image bar 36 and the mark 37 superposed and displayed on the image bar 36 are separately displayed as the image bar 36, and the marking of the mark 37 along the image bar 36 above the image bar 36. Below them, two thumbnail images 39 and the corresponding report contents 47 are displayed.

Thus, the user can read the observation image 31 and the information about a check point 42 from the report contents, and can newly attaches the information confirmed by the user from observation image 31 and the check point 42 to the contents of the report.

On the display screen 30 shown at the lower part in FIG. 8, the user can display the mark 37 at the part image bar 36, and can display the time mark at the part of the marking of the mark 37. Thus, all information about the entire mark 37 can be confirmed and compared with the report contents 47. (Seventh Embodiment)

FIGS. 9A, 9B, 9C, and 9D show examples of images displayed on the display screen of a monitor device of a work station in the capsule endoscope image filing system as the seventh embodiment not according to the present invention.

First, FIG. 9A shows a scale mark 48-1 displayed along the display of the image bar 36 or mark 37. From the leftmost to the right most along the mark display, the number of significant captured images (number of frames) is displayed by the scale up to, for example, 59860 in 5000 image units.

59860 images indicates the total number of images of the captured images in all image pickup paths from the mouth of the test subject 4 by the capsule endoscope 3.

Thus, in the first example of the present embodiment, a scale mark 48-1 indicating the entire captured images by the number of images is provided. By displaying the scale for the number of images, the position of a selected thumbnail image can be show to the user.

The user can select a thumbnail image from the scale mark 48-1 indicating the number of images, and add to the thumbnail image a label and comment including the information about the time, marking , etc.

In the display of the scale mark 48-1, at the position where there is a gap without image information, for example, a gap 49 as totally black display can be displayed for the space of the number of acquired images of estimated captured images.

Since the obtained significant images are displayed with the number of images in the order of acquisition in serial numbers, the practical amount of data can be clarified. In addition, when according to images are selected in a short time, images can be easily identified how many images are omitted between the significant images to identify the distance between the images.

The display unit of the number of images by the scale mark 48-1 is 5000 images in the example shown in FIG. 9A. However, the present invention is not limited to this application, and the user can optionally specify and change the number.

Although not shown in the attached drawings, a user can set a specific region (landmark). When a specific region is set, the number of images in the set region can be display at the position of the scale mark 48-1 corresponding to the set region.

Next, FIG. 9B shows a scale mark 48-2 displayed with the display of the image bar 36 or the mark 37. The scale mark 48-2 shows the scale of the capture time of images from the leftmost to the rightmost along the mark display in, for example, one hour (1:00:00) unit from 0 hour 00 minute 00 second to 8 hours 15 minutes 00 second.

Also in this case, when there is a gap without image information, the space corresponding to the capture time of the images estimated for the gap is displayed by total black display as the gap 49.

Thus, by displaying the scale of the elapsed time from the start of an inspection (image pickup), or the elapsed time from the position of 0 hour set by a user, the display can be used in estimating the time and the position at which the capsule endoscope 3 passes a target organ.

FIG. 9C shows a scale mark 48-3 displayed along the display of the image bar 36 or the mark 37. The scale mark 48-3 indicates a scale of 0% to 100% from the leftmost to the rightmost along the capture time axis or along the mark display in a 10% unit as a rate of the number of captured images.

Thus, by displaying the ratio to the capture time axis or the number of captured images, the relative position of the target point can be indicated with respect to the entire inspection or the feature region selected by a user.

When the relative position with respect to the entire inspection is regarded, it is easy to select the process of the next step of, for example, starting an observation from the mouth or the large intestine, etc.

The gap 49 shown in FIGS. 9A, 9B, and 9C can be, in any case, displayed as a simple gap mark 50 indicating the gap starting position as shown in the example of the scale mark 48-1 shown in, for example, FIG. 9D.

An image bar 38 is represented as a long horizontal image bar 36a by horizontally arranging a provided line in a time series, but it is obvious that the direction is not limited to the application. That is, the vertical direction (36b), the diagonal direction (36c), or the screen depth direction can be expressed in a three-dimensional array (36d).

In addition, the mark 37 is expressed by a vertical line (37a) with respect to a horizontal bar, but the arrangement is not limited to this application. A dot, a circle (37b), a square, or an application in which the length of a vertical line is different from the width of a bar (longer line (37c), or shorter line (37d)) can be used. FIG. 11 shows these examples.

In addition, a partial enlargement display 44 shown in FIG. 6 can display not only an existing available area of thumbnail images, but display a generated available area by shifting thumbnail images in a target range specified by a user.

The present invention is not limited to the above-mentioned embodiments.

## Claims

1. A display processing apparatus for displaying on a display screen image information about the video of plural pieces obtained by capturing with time the video in a plurality of positions in a body of a test subject using an image pickup apparatus provided in the body of the test subject, comprising:
an image bar generation device adapted to arrange a line expressed in an average color calculated for each piece of video or for plural pieces of video on a display screen (30) in a time series, and adapted to generate an image bar (36) that can be totally displayed as a bar;
a mark generation device adapted to generate a mark (37) for displaying a feature determined from video as a feature extracted mark extracted from the video; and
a mark display device adapted to display the mark (37) generated by the mark generation device separated in parallel above the image bar (36),
**characterized in that**:
the mark display device is further adapted to display the mark (37) superposed on the image bar (36), to switch the display of the mark (37) from the display of the mark (37) superposed and displayed on the image bar (36) when an observation image (31) is displayed, into the display of the mark (37) separated in parallel above the image bar (36) in response to a user input and in cooperation with a replacement of an area of the display screen (30) in which the observation image (31) is displayed, and from the display of the mark (37) separated in parallel above the image bar (36) when the observation image (31) is displayed, into the display of the mark (37) superposed and displayed on the image bar (36) when the user specifies a display of the observation image (31) corresponding to an enlargement of the area of the display screen (30) in which the observation image (31) is displayed.

2. The apparatus according to claim 1, wherein the line is vertical, the bar is horizontal, and the vertical line is horizontally arranged on the display screen (30).

3. The apparatus according to claim 1 or 2, wherein the enlargement results from displaying the observation image (31) as an enlarged observation image.

4. The apparatus according to claim 1 or 2, wherein the enlargement results from displaying a plurality of the observation images (31).

5. The apparatus according to claim 1 or 2, wherein the user input instructs displaying a report entry area (46), the report entry area (46) replacing the area of the display screen (30) where the observation image (31) is displayed.

6. The apparatus according to any one of the preceding claims, wherein the switch of the display of the mark (37) is automatically carried out in response to the user input or the user specification.

7. The apparatus according to any one of the preceding claims, wherein the mark (37) is a type of lesion color mark.

## Patentansprüche

1. Anzeigeverarbeitungsvorrichtung zum Anzeigen von Bildinformation über das Video aus mehreren Teilen auf einem Bildschirm, wobei die Teile durch Erfassen des Videos an mehreren Stellen in einem Körper eines Versuchssubjekts unter Verwendung einer Bildaufnahmevorrichtung gewonnen sind, die in dem Körper des Versuchssubjekts bereitgestellt ist, umfassend:
eine Bildbalkenerzeugungsvorrichtung, die dazu eingerichtet ist, eine in einer Durchschnittsfarbe wiedergegebene, für jeden Teil des Videos oder für mehrere Teile des Videos berechnete Linie auf einem Bildschirm (30) in einer Zeitfolge anzuordnen,
und dazu eingerichtet ist, einen Bildbalken (36) zu erzeugen, der vollständig als ein Balken angezeigt werden kann;
eine Markierungserzeugungsvorrichtung, die dazu eingerichtet ist, eine Markierung (37) zum Anzeigen eines aus dem Video ermittelten Merkmals als eine dem Video entnommene Merkmalsentnahmemarkierung zu erzeugen; und
eine Markierungsanzeigevorrichtung, die dazu eingerichtet ist, die von der Markierungserzeugungsvorrichtung erzeugte Markierung (37) separat parallel über dem Bildbalken (36) anzuzeigen,
**dadurch gekennzeichnet, dass** die Markierungsanzeigevorrichtung ferner dazu eingerichtet ist,
die Markierung (37) auf dem Bildbalken (36) überlagert anzuzeigen,
die Anzeige der Markierung (37), wenn ein Betrachtungsbild (31) angezeigt ist, von der Anzeige der auf dem Bildbalken (36) überlagerten und angezeigten Markierung (37) zu der Anzeige der Markierung (37) separat parallel über dem Bildbalken (36) umzuschalten in Antwort auf eine Benutzereingabe und zusammenwirkend mit einer Ersetzung eines Bereichs auf dem Bildschirm (30), in dem das Betrachtungsbild (31) angezeigt ist, und von der Anzeige der Markierung (37) separat parallel über dem Bildbalken (36), wenn das Betrachtungsbild (31) angezeigt ist, zu der auf dem Bildbalken (36) überlagerten und angezeigten Anzeige der Markierung (37) umzuschalten, wenn der Benutzer eine Anzeige des Betrachtungsbilds (31) bestimmt, die einer Vergrößerung des Bereichs auf dem Bildschirm (30) entspricht, in dem das Betrachtungsbild (31) angezeigt ist.

2. Vorrichtung nach Anspruch 1, wobei die Linie vertikal ausgerichtet, der Balken horizontal ausgerichtet und die vertikale Linie horizontal auf dem Bildschirm (30) angeordnet ist.

3. Vorrichtung nach Anspruch 1 oder 2, wobei sich die Vergrößerung aus dem Anzeigen des Betrachtungsbilds (31) als ein vergrößertes Betrachtungsbild ergibt.

4. Vorrichtung nach Anspruch 1 oder 2, wobei sich die Vergrößerung aus dem Anzeigen mehrerer Betrachtungsbilder (31) ergibt.

5. Vorrichtung nach Anspruch 1 oder 2, wobei die Benutzereingabe anweist, einen Berichtseingabebereich (46) anzuzeigen, wobei der Berichtseingabebereich (46) den Bereich auf dem Bildschirm (30) ersetzt, in dem das Betrachtungsbild (31) angezeigt ist.

6. Vorrichtung nach einem der vorangehenden Ansprüche, wobei das Schalten der Anzeige der Markierung (37) automatisch in Antwort auf die Benutzereingabe oder die Bestimmung durch den Benutzer ausgeführt wird.

7. Vorrichtung nach einem der vorangehenden Ansprüche, wobei die Markierung (37) von der Art einer verletzungsfarbigen Markierung ist.

## Revendications

1. Appareil de traitement d'affichage pour afficher sur un écran d'affichage des informations d'image relatives à la vidéo de parties multiples obtenues en capturant avec le temps la vidéo dans une pluralité de positions dans un corps d'un sujet de test en utilisant un appareil de capture d'images prévu dans le corps du sujet de test, comprenant :
un dispositif de génération de barre d'images adapté à agencer une ligne exprimée dans une couleur moyenne calculée pour chaque partie de vidéo ou pour des parties multiples de vidéo sur un écran (30) d'affichage dans une série temporelle, et adapté à générer une barre (36) d'images qui peut être totalement affichée comme une barre ;
un dispositif de génération de marque adapté à générer une marque (37) pour afficher une caractéristique déterminée à partir d'une vidéo comme une marque extraite d'une caractéristique extraite de la vidéo ; et
un dispositif d'affichage de marque adapté à afficher la marque (37) générée par le dispositif de génération de marque séparée en parallèle au-dessus de la barre (36) d'images,
**caractérisé en ce que** :
le dispositif d'affichage de marque est en outre adapté à afficher la marque (37) superposée sur la barre (36) d'images, à commuter l'affichage de la marque (37) de l'affichage de la marque (37) superposée et affichée sur la barre (36) d'images lorsqu'une image (31) d'observation est affichée, à l'affichage de la marque (37) séparée en parallèle au-dessus de la barre (36) d'images en réponse à une entrée d'utilisateur et en coopération avec un remplacement d'une zone de l'écran (30) d'affichage dans laquelle l'image (31) d'observation est affichée, et de l'affichage de la marque (37) séparée en parallèle au-dessus de la barre (36) d'images lorsque l'image (31) d'observation est affichée, à l'affichage de la marque (37) superposée et affichée sur la barre (36) d'images lorsque l'utilisateur spécifie un affi - chage de l'image (31) d'observation correspondant à un agrandissement de la zone de l'écran (30) d'affichage dans laquelle l'image (31) d'observation est affichée.

2. Appareil selon la revendication 1, dans lequel la ligne est verticale, la barre est horizontale, et la ligne verticale est agencée horizontalement sur l'écran (30) d'affichage.

3. Appareil selon la revendication 1 ou 2, dans lequel l'agrandissement résulte de l'affichage de l'image (31) d'observation comme une image d'observation agrandie.

4. Appareil selon la revendication 1 ou 2, dans lequel l'agrandissement résulte de l'affichage d'une pluralité des images (31) d'observation.

5. Appareil selon la revendication 1 ou 2, dans lequel l'entrée d'utilisateur commande d'afficher une zone (46) d'entrée de rapport, la zone (46) d'entrée de rapport remplaçant la zone de l'écran (30) d'affichage où l'image (31) d'observation est affichée.

6. Appareil selon l'une quelconque des revendications précédentes, dans lequel la commutation de l'affichage de la marque (37) est automatiquement exécutée en réponse à l'entrée d'utilisateur ou à la spécification d'utilisateur.

7. Appareil selon l'une quelconque des revendications précédentes, dans lequel la marque (37) est un type de marque en couleur de lésion.
